# EUROPEAN PATENT APPLICATION

(11) **EP 2 036 572 A1**
(43) Date of publication of application: **18.03.2009**
(21) Application number: 07115626.9
(22) Date of filing: 04.09.2007
(51) Int. Cl.: A61K 38/22, A61K 38/26, A61K 38/28, A61K 38/34, A61K 9/14

(54) **Process for drying a protein, a protein particle and a pharmaceutical composition comprising the protein particle**

(71) Applicant: Novo Nordisk A/S, 2880 Bagsværd (DK)
(72) Inventor: The designation of the inventor has not yet been filed

(57) **Abstract**

The invention concerns a process for preparing protein particles comprising a) providing a suspension of protein; and b) drying the suspension, wherein the suspension of protein is not homogenised. Further is disclosed a protein particle.

## Description

### FIELD OF THE INVENTION

The present invention relates to a process for preparing protein particles and the protein particle. The invention also relates to pharmaceutical compositions containing such protein particles, to methods of treating diabetes, hyperglycaemia or obesity using the protein particle and to the use of such protein particle for the treatment of diabetes or hyperglycaemia.

### BACKGROUND OF THE INVENTION

There is a continued interest in developing new and improved microparticles for use in the pharmaceutical industry. Further it is of high priority to develop technologies for producing microparticles which also after production show drug activity and are stable.

Production of micro particles of protein can be obtained in many ways such as by controlled crystallization or precipitation, spray drying, spray freeze drying or super critical fluid processing to produce a particle of the desired size. Alternatively production of larger primary particles followed by micronization of these particles to the desired size is widely used.

For nasal, bronchial and pulmonary drug delivery the mass median aerodynamic diameter of the particles administered determines the place of deposition. Because of the large difference in size required, unique particle formation processes often has to be developed if a drug has to be tested for more than one route of delivery. E.g. for pulmonary delivery a mass mean aerodynamic diameter (MMAD) of 1-5 µm is normally considered optimal and for nasal delivery MMAD must be above 10 µm. For products intended for intravenous administration, it is desirable to make particles less than 6um in size.

US 6652837 concerns particles for drug delivery to the pulmonary system, where the particles consists of a therapeutic agent and excipients such as a surfactant. Examples are given on spray drying of a solution of insulin particles with excipients.

International patent application WO 03/015756 discloses a method for producing microparticles of a particle forming material comprising the steps of a) forming a suspension of the particle forming material and b) spray-drying said suspension. The formation of a suspension of the particle-forming material is preferably carried out by first dissolving the particle-forming material in a solvent, and then adding to the solution so formed a non-solvent for the particle-forming material, so as to bring about precipitation of the particle-forming material.

However the known methods for producing particles have a number of disadvantages such as difficulties in obtaining the desired particle size distribution and aerodynamic diameter, denaturation of the protein, collapsing of the protein particles and particles having a low density.

The present invention overcomes the problems of the prior art.

### SUMMARY OF THE INVENTION

The present invention concerns a process for preparing protein particles comprising
a) Providing a suspension of protein; and
b) drying the suspension
   wherein the suspension of protein is not homogenised.
In one aspect of the invention the invention concerns a protein particle produced by the inventive process. In one aspect the invention concerns a dry protein particle having a bulk density above about 0.2 g/cm³. In one aspect the invention concerns a pharmaceutical composition comprising the protein particle and use of such composition.

### DESCRIPTION OF THE DRAWINGS

Figure 1: Scanning electron microscopy (SEM) picture of the particles obtained by spray drying in Example 1. The length bar represents 10 µm.
Figure 2: Scanning electron microscopy (SEM) picture of two particles obtained by spray drying in Example 1. The length bar represents 1 µm.

### DEFINITIONS

The term **"suspension of protein"** or **"protein suspension"** means a suspension liquid comprising a protein in suspension.

The term **"suspension liquid"** means a liquid, wherein the drug can be suspended. That is a liquid, wherein the drug is not soluble or wherein the solubility of the drug is very low that is below 1,0 g per 100 ml of the liquid at 20°C. The suspension liquid can comprise volatile and non volatile liquids.

By **"volatile liquid"** is meant a liquid, which to some extend will evaporate upon heating and/or at reduced pressure, e.g. liquids which have a vapour pressure above 65 Pa at room temperature or an aqueous azeotropic mixture including a base having a vapour pressure above 65 Pa at room temperature.

A **"non volatile liquid"** as mentioned herein means a liquid, which do not evaporate or only partly evaporate upon heating, e.g. liquids with a vapour pressure below 65 Pa.

With **"insulin"** as used herein is meant human insulin, porcine insulin or bovine insulin with disulfide bridges between CysA7 and CysB7 and between CysA20 and CysB19 and an internal disulfide bridge between CysA6 and CysA11 or an insulin analogue or derivative thereof.

By **"analogue"** as used herein is meant a polypeptide which has a molecular structure which formally can be derived from the structure of the protein, by deleting and/or exchanging at least one amino acid residue occurring in the naturally occurring protein and/or adding at least one amino acid residue. The added and/or exchanged amino acid residues can either be codable amino acid residues or other naturally occurring residues or purely synthetic amino acid residues, The insulin analogues may be such wherein position 28 of the B chain may be modified from the natural Pro residue to one of Asp, Lys, or Ile. In another aspect Lys at position B29 of insulin is modified to Pro or Glu. Also, Asn at position A21 may be modified to Ala, Gln, Glu, Gly, His, Ile, Leu, Met, Ser, Thr, Trp, Tyr or Val, in particular to Gly, Ala, Ser, or Thr and preferably to Gly. Furthermore, Asn at position B3 may be modified to Lys,Thr, Ser, Gln, Glu or Asp. Further examples of insulin analogues are des(B30) human insulin; des(B30) human insulin analogues; insulin analogues wherein PheB1 has been deleted; insulin analogues wherein the A-chain and/or the B-chain have an N-terminal extension and insulin analogues wherein the A-chain and/or the B-chain have a C-terminal extension. Thus one or two Arg may be added to position B1. The GLP-1 analogues may be such wherein the naturally occurring Lys at position 34 of GLP-1 (7-37) has been substituted with Arg. All amino acids for which the optical isomer is not stated is to be understood to mean the L-isomer.
In aspects of the invention a maximum of 17 amino acids have been modified. In aspects of the invention a maximum of 15 amino acids have been modified. In aspects of the invention a maximum of 10 amino acids have been modified. In aspects of the invention a maximum of 8 amino acids have been modified. In aspects of the invention a maximum of 7 amino acids have been modified. In aspects of the invention a maximum of 6 amino acids have been modified. In aspects of the invention a maximum of 5 amino acids have been modified. In aspects of the invention a maximum of 4 amino acids have been modified. In aspects of the invention a maximum of 3 amino acids have been modified. In aspects of the invention a maximum of 2 amino acids have been modified. In aspects of the invention 1 amino acid has been modified.

With **"desB30 insulin", "desB30 human insulin"** is meant insulin or an analogue thereof lacking the B30 amino acid residue.

By **"parent insulin"** is meant a naturally occurring insulin such as human insulin or porcine insulin. Alternatively, the parent insulin can be an insulin analogue.

By **"derivative"** as used herein is meant a naturally occurring protein or an analogue thereof which has been chemically modified, e.g. by introducing a substituent in one or more positions of the protein backbone or by oxidizing or reducing groups of the amino acid residues in the protein or by acylating a free amino group or a hydroxy group.

### DESCRIPTION OF THE INVENTION

The invention provides a method by which particles of a given size can be designed by drying a suspension of smaller particles without homogenising the particles before drying and using the settings of the drying process to determine the final particle size. The particles do not collapse during the drying process and they are compact, dense yet porous giving a large specific surface area. Further the particles keep their biological activity after production by the inventive method.

During dissolution after deposition at the place of interest, the particles of this invention will decompose to small particles which also will have a large specific surface area and, hence, a high dissolution rate.

Furthermore, composite particles containing more than 1 active pharmaceutical ingredinent can readily be prepared by spray drying co-suspensions of multiple species of small particles.
The invention concerns a process for preparing protein particles comprising
a) Providing a suspension of protein; and
b) drying the suspension
wherein the suspension of protein is not homogenised.
In one aspect of the invention the suspension is provided and/or fed to the drying equipment at a temperature below about 8°C, below about 6°C, below about 5°C, below about 4°C, below about 3°C, below about 2°C or below about 1°C.
The solubility of most proteins tends to decrease with decreasing temperatures. To provide a suspension of a protein it is possible to lower the temperature of the suspension liquid and thereby increasing the quantity of the suspended protein. In one aspect of the invention the protein suspension is provided at a temperature below about 8°C, below about 6°C, below about 5°C, below about 4°C, below about 3°C, below about 2°C or below about 1°C.
In one aspect of the invention the protein suspension is stored at a temperature below about 8°C, below about 6°C, below about 5°C, below about 4°C, below about 3°C, below about 2°C or below about 1°C.
In one aspect of the invention the equipment for feeding the suspension into the dryer including the feeding tank, pipelines and the feeding nozzles are cooled to a temperature below about 8°C, below about 6°C, below about 5°C, below about 4°C, below about 3°C, below about 2°C or below about 1°C.
In one aspect the freezing point of the suspension liquid is depressed and a protein suspension is obtained at a temperature below 0°C. In one aspect the freezing point of the suspension is depressed and the protein suspension is stored and fed at a temperature below 0°C.

The invention will be summarized in the following paragraphs:
1. A process for preparing protein particles comprising
   a) Providing a suspension of protein; and
   b) drying the suspension
   wherein the suspension of protein is not homogenised.
2. A process according to paragraph 1, wherein the suspension is provided and/or fed to the drying equipment at a temperature below about 8°C, below about 6°C, below about 5°C, below about 4°C, below about 3°C, below about 2°C or below about 1°C.
3. A process according to paragraphs 1-2, wherein the suspension comprises a volatile liquid and/or a non volatile liquid.
4. A process according to any of the preceding paragraphs, wherein the drying method for drying the suspension is selected from the group consisting of: spray drying, spray freeze drying, fluid bed drying and freeze drying.
5. A process according to any of the preceding paragraphs, wherein at least 50% of the protein particles has a geometric diameter in the range of 1-100 µm.
6. A process according to any of the preceding paragraphs, wherein at least 70% of the protein particles has a geometric diameter in the range of 1-100 µm.
7. A process according to any of the preceding paragraphs, wherein at least 90% of the protein particles has a geometric diameter in the range of 1-100 µm.
8. A process according to any of the preceding paragraphs, wherein at least 50% of the protein particles has a geometric diameter in the range of 1-50 µm.
9. A process according to any of the preceding paragraphs, wherein at least 70% of the protein particles has a geometric diameter in the range of 1-50 µm.
10. A process according to any of the preceding paragraphs, wherein at least 90% of the protein particles has a geometric diameter in the range of 1-50 µm.
11. A process according to any of the preceding paragraphs, wherein at least 50% of the protein particles has a geometric diameter in the range of 1-20 µm.
12. A process according to any of the preceding paragraphs, wherein at least 70% of the protein particles has a geometric diameter in the range of 1-20 µm.
13. A process according to any of the preceding paragraphs, wherein at least 90% of the protein particles has a geometric diameter in the range of 1-20 µm.
14. A process according to any of the preceding paragraphs, wherein at least 50% of the protein particles has a geometric diameter in the range of 1-10 µm.
15. A process according to any of the preceding paragraphs, wherein at least 70% of the protein particles has a geometric diameter in the range of 1-10 µm.
16. A process according to any of the preceding paragraphs, wherein at least 90% of the protein particles has a geometric diameter in the range of 1-10 µm.
17. A process according to any of the preceding paragraphs, wherein at least 50% of the protein particles has a geometric diameter in the range of 1-5 µm, for example 1-3 µm.
18. A process according to any of the preceding paragraphs, wherein at least 70% of the protein particles has a geometric diameter in the range of 1-5 µm, for example 1-3 µm.
19. A process according to any of the preceding paragraphs, wherein at least 90% of the protein particles has a geometric diameter in the range of 1-5 µm, for example 1-3 µm.
20. A process according to any of the preceding paragraphs, wherein at least 92% of the protein particles has a geometric diameter in the range of 1-5 µm, for example 1-3 µm.
21. A process according to any of the preceding paragraphs, wherein at least 95% of the protein particles has a geometric diameter in the range of 1-5 µm, for example 1-3 µm.
22. A process according to paragraphs 1-10, wherein at least 50% of the protein particles has a geometric diameter in the range of 10-50 µm, for example 10-25 µm.
23. A process according to paragraphs 1-10 and 22, wherein at least 70% of the protein particles has a geometric diameter in the range of 10-50 µm, for example 10-25 µm.
24. A process according to paragraphs 1-10 and 22-23, wherein at least 90% of the protein particles has a geometric diameter in the range of 10-50 µm, for example 10-25 µm.
25. A process according to paragraphs 1-10 and 22-24, wherein at least 92% of the protein particles has a geometric diameter in the range of 10-50 µm, for example 10-25 µm.
26. A process according to paragraphs 1-10 and 22-25, wherein at least 95% of the protein particles has a geometric diameter in the range of 10-50 µm, for example 10-25 µm.
27. A process according to any of the preceding paragraphs, wherein the protein is selected from the group consisting of insulin, insulin analogues, insulin derivatives, GLP-1, GLP-1 analogues, GLP-1 derivatives, glucagon, glucagon analogues, glucagon derivatives, exendin, exendin analogues and derivatives, amylin, amylin analogues, amylin derivatives, α-MSH, α -MSH analogues, α -MSH derivatives and/or any combination thereof.
28. A process according to any of the preceding paragraphs, wherein the insulin analogue is selected from the group consisting of AspB28 human insulin, LysB28ProB29 human insulin, GlyA21ArgB31ArgB32 human insulin, LysB3GluB29 human insulin.
29. A process according to paragraphs 1 and 27-28 wherein the suspension comprises zinc.
30. A process according to any of the preceding paragraphs, wherein the suspension comprises more than one protein.
31. A process according to any of the preceding paragraphs, wherein the suspension comprises an insulin analogue and an insulin derivative.
32. A process according to any of the preceding paragraphs, wherein the suspension comprises at least one protein dissolved in the suspension.
33. A process according to any of the preceding paragraphs, wherein the suspension comprises an excipients selected from the group consisting of: a buffer, a detergent, a stabilizer, a protease inhibitor, a flavour, a carrier, an absorption protracting agent, a bulking agent or an agent improving the flowing properties or a penetration enhancer or a combination hereof.
34. A process according to any of the preceding paragraphs, wherein the excipients are suspended in the suspension.
35. A process according to any of the preceding paragraphs, wherein the excipients are dissolved in the suspension.
36. A process according to any of the preceding paragraphs, wherein the suspension liquid is an organic solvent selected from the group of methanol, ethanol, ethyl acetate, acetone, acetonitrile, butanol, butyl acetate, chloroform, cyclohexane, heptane, tetrahydrofuran, tetrafluoroethane and any combination thereof.
37. A process according to any of the preceding paragraphs, wherein the suspension liquid is ethanol.
38. A process according to any of the preceding paragraphs, wherein the suspension liquid is water.
39. A process according to any of the preceding paragraphs, wherein the protein particles is dried to liquid content below about 10%, below about 6%, below about 4%, below about 2% or below about 1 %.
40. A process according to any of the preceding paragraphs, wherein the protein particles has an activity of at least 40% of the activity of the suspended protein.
41. A process according to any of the preceding paragraphs, wherein the protein particles has an activity of at least 50% of the activity of the suspended protein, at least 60% of the activity of the suspended protein, at least 65% of the activity of the suspended protein, at least 70% of the activity of the suspended protein, at least 75% of the activity of the suspended protein, at least 80% of the activity of the suspended protein, at least 85% of the activity of the suspended protein, at least 90% of the activity of the suspended protein, at least 95% of the activity of the suspended protein or at least 98% of the activity of the suspended protein.
42. A protein particle produced by the process of paragraphs 1-41.
43. A dry protein particle having a bulk density above about 0.2 g/cm³.
44. A protein particle according to paragraph 43 having a bulk density between about 0.2 g/cm³ and about 0.5 g/cm³.
45. A protein particle according to paragraphs 43-44 having a bulk density between about 0.25 g/cm³ and about 0.45 g/cm³.
46. A protein particle according to paragraphs 43-45 having a bulk density between about 0.3 g/cm³ and about 0.4 g/cm³.
47. A protein particle according to paragraphs 43-46 having a bulk density between about 0.3 g/cm³ and about 0.35 g/cm³.
48. A protein particle according to paragraphs 43-47 having a tap density between about 0.1 g/cm³ and about 0.6 g/cm³.
49. A protein particle according to paragraphs 43-48 having a tap density between about 0.1 g/cm³ and about 0.5 g/cm³.
50. A protein particle according to paragraphs 43-49 having a tap density between about 0.15 g/cm³ and about 0.3 g/cm³.
51. A protein particle according to paragraphs 43-50 having a tap density about 0.2 g/cm³.
52. A protein particle according to paragraphs 43-51 having an aerodynamic diameter between about 1 µm and about 5 µm.
53. A protein particle according to paragraphs 43-52 having an aerodynamic diameter between about 2 µm and about 4 µm.
54. A protein particle according to paragraphs 43-53 having an aerodynamic diameter between about 2 µm and about 3 µm.
55. A protein particle according to paragraphs 43-54, wherein at least 50% of the protein particles has a geometric diameter in the range of 1-100 µm.
56. A protein particle according to paragraphs 43-55, wherein at least 70% of the protein particles has a geometric diameter in the range of 1-100 µm.
57. A protein particle according to paragraphs 43-56, wherein at least 90% of the protein particles has a geometric diameter in the range of 1-100 µm.
58. A protein particle according to paragraphs 43-57, wherein at least 50% of the protein particles has a geometric diameter in the range of 1-50 µm.
59. A protein particle according to paragraphs 43-58, wherein at least 70% of the protein particles has a geometric diameter in the range of 1-50 µm.
60. A protein particle according to paragraphs 43-59, wherein at least 90% of the protein particles has a geometric diameter in the range of 1-50 µm.
61. A protein particle according to paragraphs 43-60, wherein at least 50% of the protein particles has a geometric diameter in the range of 1-20 µm.
62. A protein particle according to paragraphs 43-61, wherein at least 70% of the protein particles has a geometric diameter in the range of 1-20 µm.
63. A protein particle according to paragraphs 43-62, wherein at least 90% of the protein particles has a geometric diameter in the range of 1-20 µm.
64. A protein particle according to paragraphs 43-63, wherein at least 50% of the protein particles has a geometric diameter in the range of 1-10 µm.
65. A protein particle according to paragraphs 43-64, wherein at least 70% of the protein particles has a geometric diameter in the range of 1-10 µm.
66. A protein particle according to paragraphs 43-65, wherein at least 90% of the protein particles has a geometric diameter in the range of 1-10 µm.
67. A protein particle according to paragraphs 43-66, wherein at least 50% of the protein particles has a geometric diameter in the range of 1-5 µm, for example 1-3 µm.
68. A protein particle according to paragraphs 43-67, wherein at least 70% of the protein particles has a geometric diameter in the range of 1-5 µm, for example 1-3 µm.
69. A protein particle according to paragraphs 43-68, wherein at least 90% of the protein particles has a geometric diameter in the range of 1-5 µm, for example 1-3 µm.
70. A protein particle according to paragraphs 43-69, wherein at least 92% of the protein particles has a geometric diameter in the range of 1-5 µm, for example 1-3 µm.
71. A protein particle according to paragraphs 43-70, wherein at least 96% of the protein particles has a geometric diameter in the range of 1-5 µm, for example 1-3 µm.
72. A protein particle according to paragraphs 43-50, wherein at least 50% of the protein particles has a geometric diameter in the range of 10-50 µm, for example 10-25 µm.
73. A protein particle according to paragraphs 43-50 and 72, wherein at least 70% of the protein particles has a geometric diameter in the range of 10-50 µm, for example 10-25 µm.
74. A protein particle according to paragraphs 43-50 and 72-73, wherein at least 90% of the protein particles has a geometric diameter in the range of 10-50 µm, for example 10-25 µm.
75. A protein particle according to paragraphs 43-50 and 72-74, wherein at least 92% of the protein particles has a geometric diameter in the range of 10-50 µm, for example 10-25 µm.
76. A protein particle according to paragraphs 43-50 and 72-75, wherein at least 95% of the protein particles has a geometric diameter in the range of 10-50 µm, for example 10-25 µm.
77. A protein particle according to paragraphs 43-76, wherein the protein is selected from the group consisting of insulin, insulin analogues, insulin derivatives, GLP-1, GLP-1 analogues, GLP-1 derivatives, glucagon, glucagon analogues, glucagon derivatives, exendin, exendin analogues and derivatives, amylin, amylin analogues, amylin derivatives, α-MSH, α - MSH analogues, α -MSH derivatives and/or any combination thereof.
78. A protein particle according to paragraphs 73-77, wherein the insulin analogue is selected from the group consisting of AspB28 human insulin, LysB28ProB29 human insulin, GlyA21ArgB31ArgB32 human insulin, LysB3GluB29 human insulin.
79. A protein particle according to paragraphs 43-78, wherein the particle comprises zinc.
80. A protein particle according to paragraphs 43-79, wherein the particles has a liquid content below about 10%, below about 6%, below about 4%, below about 2% or below about 1%.
81. Pharmaceutical composition comprising a therapeutically effective amount of a protein particle according to paragraphs 43-80.
82. Pharmaceutical composition according to paragraph 81, wherein the composition is a dry powder.
83. Pharmaceutical composition according to paragraphs 80-82, wherein the composition is for pulmonary, parental, nasal or oral use.
84. Pharmaceutical composition according to paragraphs 81-83 for the treatment of diabetes or hyperglycaemia in a patient in need of such treatment, comprising a therapeutically effective amount of the pharmaceutical composition, wherein the protein is selected from the group consisting of insulin, insulin analogues, insulin derivatives, GLP-1, GLP-1 analogues, GLP-1 derivatives, glucagon, glucagon analogues, glucagon derivatives, exendin, exendin analogues and derivatives, amylin, amylin analogues, amylin derivatives, α-MSH, α -MSH analogues, α -MSH derivatives and/or any combination thereof.
85. Pharmaceutical composition according to paragraphs 81-84 for the treatment of obesity or preventing overweight in a patient in need of such treatment, comprising a therapeutically effective amount of the pharmaceutical composition, wherein the protein is selected from the group consisting of α-MSH, α -MSH analogues, α -MSH derivatives and/or any combination thereof.
86. A method of treating diabetes in a patient in need of such a treatment by the use of a therapeutically effective amount of a protein particle according to paragraphs 43-80 or a pharmaceutical composition according to paragraphs 81-84.
87. A method of treating obesity or preventing overweight in a patient in need of such treatment, comprising a therapeutically effective amount of a protein particle according to paragraphs 43-80 or a pharmaceutical composition according to paragraphs 81-85, wherein the protein is selected from the group consisting of α-MSH, α -MSH analogues, α -MSH derivatives and/or any combination thereof.
88. Use of a protein particle according to paragraphs 43-80 or a pharmaceutical composition according to paragraphs 81-84 for the treatment of diabetes or hyperglycaemia in a patient in need of such treatment.
89. Use according to paragraph 88, wherein the protein is selected from the group consisting of insulin, insulin analogues, insulin derivatives, GLP-1, GLP-1 analogues, GLP-1 derivatives, glucagon, glucagon analogues, glucagon derivatives, exendin, exendin analogues and derivatives, amylin, amylin analogues, amylin derivatives, α-MSH, α -MSH analogues, α - MSH derivatives and/or any combination thereof.
90. Use according to paragraphs 88-89, wherein the protein particle is administered pulmonally.
91. Protein particle as described in the examples.

### PHARMACEUTICAL COMPOSITIONS

When preparing the insulin to be used for preparing protein particles according to the invention, it can be produced by either well-know peptide synthesis or by well known recombinant production in suitable transformed microorganisms. Thus the insulin starting product can be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture. Insulin derivatives to be used for preparing protein particles according to the invention can be prepared by using the above mentioned insulin as the starting product for the substitution. Reference is made to international patent application WO 2005/012347.

Further protein particles to be dried according to the invention can be prepared using a supercritical fluid with enhanced mixing. Reference is made to international patent application WO 2004/071614.

The protein particle of this invention can, for example, be administered subcutaneously, orally, nasally or pulmonary.

For subcutaneous administration, the protein particle is formulated analogously with the formulation of known protein particles, the physicians are familiar with this procedure.

The protein particle of this invention may be administered by inhalation in a dose effective manner to increase circulating protein levels for example to increase the level of insulin in order to lower circulating glucose levels. Such administration can be effective for treating disorders such as diabetes or hyperglycaemia. Achieving effective doses of protein particles requires administration of an inhaled dose of protein particles of this invention of more than about 0.5 µg/kg to about 50 µg/kg. A therapeutically effective amount can be determined by a knowledgeable practitioner, who will take into account factors for example including insulin level, blood glucose levels, the physical condition of the patient, the patient's pulmonary status, or the like.

According to the invention, protein particles of this invention may be delivered by inhalation to achieve rapid absorption thereof. Administration by inhalation can result in pharmacokinetics comparable to subcutaneous administration of insulin. Inhalation of protein particles of this invention leads to a rapid rise in the level of circulating protein, for example when insulin is inhaled the inhalation is followed by a rapid fall in blood glucose levels. Different inhalation devices typically provide similar pharmacokinetics when similar particle sizes and similar levels of lung deposition are compared.

According to the invention, protein particles of this invention may be delivered by any of a variety of inhalation devices known in the art for administration of a therapeutic agent by inhalation. These devices include metered dose inhalers, nebulizers, dry powder generators, sprayers, and the like. Protein particles of this invention is delivered by a dry powder inhaler or a sprayer. There are a several desirable features of an inhalation device for administering protein particles of this invention. For example, delivery by the inhalation device is advantageously reliable, reproducible, and accurate. The inhalation device should deliver small particles, for example particles less than about 10 µm, for example about 1-5 µm, for good respirability. Some specific examples of commercially available inhalation devices suitable for the practice of this invention are Turbohaler^{™} (Astra), Rotahaler^{®} (Glaxo), Diskus^{®} (Glaxo), Spiros^{™} inhaler (Dura), devices marketed by Inhale Therapeutics, AERx^{™} (Aradigm), the Ultravent^{®} nebulizer (Mallinckrodt), the Acorn II^{®} nebulizer (Marquest Medical Products), the Ventolin^{®} metered dose inhaler (Glaxo), the Spinhaler^{®} powder inhaler (Fisons), Cytclohaler ^{™} or the like.

As those skilled in the art will recognize, the formulation of protein particles of the invention, the quantity of the formulation delivered, and the duration of administration of a single dose depend on the type of inhalation device employed. For some aerosol delivery systems, such as nebulizers, the frequency of administration and length of time for which the system is activated will depend mainly on the concentration of insulin conjugate in the aerosol. For example, shorter periods of administration can be used at higher concentrations of insulin conjugate in the nebulizer solution. Devices such as metered dose inhalers can produce higher aerosol concentrations, and can be operated for shorter periods to deliver the desired amount of protein. Devices such as powder inhalers deliver active agent until a given charge of agent is expelled from the device. In this type of inhaler, the amount of protein particles of this invention in a given quantity of the powder determines the dose delivered in a single administration.

The particle size of the protein particles in the formulation delivered by the inhalation device is critical with respect to the ability of protein particles to make it into the lungs, and into the lower airways or alveoli. The protein particles of this invention can be formulated so that at least about 10% of the protein particles delivered are deposited in the lung, for example about 10 to about 20%, or more. It is known that the maximum efficiency of pulmonary deposition for mouth breathing humans is obtained with particle sizes of about 2 µm to about 3 µm. Pulmonary deposition decreases substantially when particle sizes are above about 5 µm. Particle sizes below about 1 µm cause pulmonary deposition to decrease, and it becomes difficult to deliver particles with sufficient mass to be therapeutically effective. Thus, protein particles delivered by inhalation have a particle size less than about 10 µm, for example in the range of about 1 µm to about 5 µm. The formulation of protein particles is selected to yield the desired particle size in the chosen inhalation device.

Advantageously for administration as a dry powder, protein particles of this invention is prepared in a particulate form with a particle size of less than about 10 µm, for example about 1 to about 5 µm. The particle size is effective for delivery to the alveoli of the patient's lung. The dry powder is largely composed of particles produced so that a majority of the particles have a size in the desired range. Advantageously, at least about 50% of the dry powder is made of particles having a diameter less than about 10 µm.

The particles are usually separated from a dry powder formulation in a container and then transported into the lung of a patient via a carrier air stream. Typically, in current dry powder inhalers, the force for breaking up the solid is provided solely by the patient's inhalation. In another type of inhaler, air flow generated by the patient's inhalation activates an impeller motor which deagglomerates the particles.

Formulations of protein particles of this invention for administration from a dry powder inhaler typically include a finely divided dry powder containing the protein particle, but the powder can also include a bulking agent, carrier, excipients, additives or the like. Additives can be included in a dry powder formulation of protein particles, for example, to dilute the protein particles as required for delivery from the particular powder inhaler, to facilitate processing of the formulation, to provide advantageous powder properties to the formulation, to facilitate dispersion of the powder from the inhalation device, to stabilize the formulation (for example, antioxidants or buffers), to provide taste to the formulation, or the like. Advantageously, the additive does not adversely affect the patient's airways. The protein particles can be mixed with an additive at a molecular level or the solid formulation can include particles of the protein conjugate mixed with or coated on particles of the additive. Typical additives include mono-, di-, and polysaccharides; sugar alcohols and other polyols, such as, for example, lactose, glucose, raffinose, melezitose, lactitol, maltitol, trehalose, sucrose, mannitol, starch, or combinations thereof; surfactants, such as sorbitols, diphosphatidyl choline, or lecithin; or the like. Typically an additive, such as a bulking agent, is present in an amount effective for a purpose described above, often at about 50% to about 99% by weight of the formulation.

The protein suspension may optionally be combined with pharmaceutical carriers or excipients which are suitable for respiratory and pulmonary administration. Such carriers may serve simply as bulking agents when it is desired to reduce the protein concentration in the powder which is being delivered to a patient, but may also serve to enhance the stability of the protein compositions and to improve the dispersability of the powder within a powder dispersion device in order to provide more efficient and reproducible delivery of the protein and to improve handling characteristics of the protein such as flowability and consistency to facilitate manufacturing and powder filling.

Suitable carrier materials may be in the form of an amorphous powder, a crystalline powder, or a combination of amorphous and crystalline powders. Suitable materials include carbohydrates, e.g., monosaccharides such as fructose, galactose, glucose, D-mannose, sorbose, and the like; disaccharides, such as lactose, trehalose, cellobiose, and the like; cyclodextrins, such as 2-hydroxypropyl--cyclodextrin; and polysaccharides, such as raffinose, maltodextrins, dextrans, and the like; (b) amino acids, such as glycine, arginine, aspartic acid, glutamic acid, cysteine, lysine, and the like; (c) organic salts prepared from organic acids and bases, such as sodium citrate, sodium ascorbate, magnesium gluconate, sodium gluconate, tromethamine hydrochloride, and the like; (d) peptides and proteins, such as aspartame, human serum albumin, gelatin, and the like; (e) alditols, such as mannitol, xylitol, and the like. A preferred group of carriers includes lactose, trehalose, raffinose, maltodextrins, glycine, sodium citrate, tromethamine hydrochloride, human serum albumin, and mannitol.

Such carrier materials may be combined with the protein suspension prior to drying, i.e., by adding the carrier material to the protein suspension which is prepared for drying. In that way, the carrier material will be formed simultaneously with and as part of the protein particles.

Typically, when the carrier is formed by drying together with the protein, the protein will be present in each individual particle at a weight percent in the range from 5% to 95%, preferably from 20% to 80%. The remainder of the particle will primarily be carrier material (typically being from 5% to 95%, usually being from 20% to 80% by weight), but will also include buffer (s) may include other components as described above.

Alternatively, the carriers may be separately prepared in a dry powder form and combined with the protein particles by blending. The separately prepared powder carriers will usually be crystalline (to avoid water absorption), but might in some cases be amorphous or mixtures of crystalline and amorphous. The size of the carrier particles may be selected to improve the flowability of the protein particles, typically being in the range from 25 to 100 µm. Carrier particles in this size range will generally not penetrate into the alveolar region of the lung and will often separate from the protein in the delivery device prior to inhalation. Thus, the particles which penetrate into the alveolar region of the lung will consist essentially of protein and buffer. A preferred carrier material is crystalline mannitol having a size in the above-stated range.

The dry protein particles of the present inventions may also be combined with other active components. For example, it may be desirable to combine small amounts of amylin or active amylin analogues protein particles comprising insulin to improve the treatment of diabetes. Amylin is a hormone which is secreted with insulin from the pancreatic 0-cells in normal (non-diabetic) individuals. It is believed that amylin modulates insulin activity in vivo, and it has been proposed that simultaneous administration of amylin with insulin could improve blood glucose control. Combining dry powder amylin with insulin in the compositions of the present invention will provide a particularly convenient product for achieving such simultaneous administration. Amylin may be combined with insulin at from 0.1 % by weight to 10% by weight (based on the total weight of insulin in a dose), preferably from 0.5% by weight to 2.5% by weight. Amylin is available from commercial suppliers, such as Amylin Corporation, San Diego, California, and can be readily formulated in the compositions of the present invention. For example, amylin may be dissolved in aqueous or other suitable solutions together with the insulin, and optionally carriers, and the solution spray dried to produce the powder product.

Formulations of protein particles of this invention suitable for use with a sprayer will typically include proteins in an aqueous solution at a concentration of about 1 mg to about 100 mg of protein particles per ml of solution. The formulation can include agents such as excipients, a buffer, an isotonicity agent, a preservative, a surfactant, and, for example zinc. The formulation can also include excipients or agents for stabilization of the protein particles , such as a buffer, a reducing agent, a bulk protein, or a carbohydrate. Bulk proteins useful in formulating protein particle conjugates include albumin, protamine, or the like. Typical carbohydrates useful in formulating protein conjugates include sucrose, mannitol, lactose, trehalose, glucose, or the like. The formulation can also include a surfactant, which can reduce or prevent surface-induced aggregation of the protein particles caused by atomization of the solution in forming an aerosol. Various conventional surfactants can be employed, such as polyoxyethylene fatty acid esters and alcohols, and polyoxyethylene sorbitol fatty acid esters. Amounts will generally range between about 0.001 and about 4% by weight of the formulation.

Pharmaceutial compositions containing a protein particle according to the present invention may also be administered nasally. The pharmaceutical composition may be administered as a liquid composition, a dry composition or a gel. For drug delivery via the nose the protein particles may be above 10 µm in order to secure deposition in the nasal cavity and to avoid that the particles are carried further down to the tracheobronchial and pulmonary region. There is no clear understanding of a upper size limit, but there probably is an upper particle size above which particles for a number of reasons will not demonstrate efficacy and maybe even could lead to local irritation.

Pharmaceutical compositions containing a protein particle according to the present invention may also be administered parental to patients in need of such a treatment. Parental administration may be performed by subcutaneous, intramuscular or intravenous injection by means of a syringe, optionally a pen-like syringe. Alternatively, parental administration can be performed by means of an infusion pump.

Injectable compositions of the protein particle of the invention can be prepared using the conventional techniques of the pharmaceutical industry which involve dissolving and mixing the ingredients as appropriate to give the desired end product. Thus, according to one procedure, protein particles according to the invention is dissolved in an amount of water which is somewhat less than the final volume of the composition to be prepared. An isotonic agent, a preservative and a buffer is added as required and the pH value of the solution is adjusted - if necessary - using an acid, e.g. hydrochloric acid, or a base, e.g. aqueous sodium hydroxide as needed. Finally, the volume of the solution is adjusted with water to give the desired concentration of the ingredients.

In a further aspect of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative aspect of the invention.

In a further aspect of the invention the formulation further comprises a pharmaceutically acceptable preservative which may be selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further aspect of the invention the preservative is present in a concentration from 0.1 mg/ml to 20 mg/ml. In a further aspect of the invention the preservative is present in a concentration from 0.1 mg/ml to 5 mg/ml. In a further aspect of the invention the preservative is present in a concentration from 5 mg/ml to 10 mg/ml. In a further aspect of the invention the preservative is present in a concentration from 10 mg/ml to 20 mg/ml. Each one of these specific preservatives constitutes an alternative aspect of the invention. The use of a preservative in pharmaceutical compositions is well-known to the skilled person. For convenience reference is made to Remington: *The Science and Practice* of *Pharmacy,* 19^{th} edition, 1995.

In a further aspect of the invention the formulation further comprises an isotonic agent which may be selected from the group consisting of a salt (e.g. sodium chloride), a sugar or sugar alcohol, an amino acid (e.g. glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one aspect the sugar additive is sucrose. Sugar alcohol is defined as a C4-C8 hydrocarbon having at least one --OH group and includes, for example, mannitol, sorbitol, inositol, galactitol, dulcitol, xylitol, and arabitol. In one aspect the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one aspect, the sugar or sugar alcohol concentration is between about 1 mg/ml and about 150 mg/ml. In a further aspect of the invention the isotonic agent is present in a concentration from 1 mg/ml to 50 mg/ml. In a further aspect of the invention the isotonic agent is present in a concentration from 1 mg/ml to 7 mg/ml. In a further aspect of the invention the isotonic agent is present in a concentration from 8 mg/ml to 24 mg/ml. In a further aspect of the invention the isotonic agent is present in a concentration from 25 mg/ml to 50 mg/ml. Each one of these specific isotonic agents constitutes an alternative aspect of the invention. The use of an isotonic agent in pharmaceutical compositions is well-known to the skilled person. For convenience reference is made to Remington: The Science and Practice of Pharmacy, 19th edition, 1995.

Typical isotonic agents are sodium chloride, mannitol, dimethyl sulfone and glycerol and typical preservatives are phenol, m-cresol, methyl p-hydroxybenzoate and benzyl alcohol.

Examples of suitable buffers are sodium acetate, glycylglycine, HEPES (4-(2-hydroxyethyl)-1-piperazineethanesulfonic acid) and sodium phosphate.

Compositions comprising protein particles of this invention, such as insulin, insulin analogues, insulin derivatives, GLP-1, GLP-1 analogues, GLP-1 derivatives, glucagon, glucagon analogues, glucagon derivatives, exendin, exendin analogues and derivatives, amylin, amylin analogues, amylin derivatives, α-MSH, α -MSH analogues and α -MSH derivatives can be used in the treatment of states which are sensitive to insulin. Thus, they can be used in the treatment of type 1 diabetes, type 2 diabetes and hyperglycaemia for example as sometimes seen in seriously injured persons and persons who have undergone major surgery. The optimal dose level for any patient will depend on a variety of factors including the efficacy of the specific insulin employed, the age, body weight, physical activity, and diet of the patient, on a possible combination with other drugs, and on the severity of the state to be treated. It is recommended that the daily dosage of the protein particle of this invention be determined for each individual patient by those skilled in the art in a similar way as for known pharmaceutical compositions.

Where expedient, the protein particles of this invention may be used in mixture with other types of proteins, e.g. a protracted insulin combined with insulin analogues with a more rapid onset of action. Examples of such insulin analogues are described e.g. in the European patent applications having the publication Nos. EP 214826 (Novo Nordisk A/S), EP 375437 (Novo Nordisk A/S) and EP 383472 (Eli Lilly & Co.).

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

### EXAMPLES

### Insulin content and purity determinations

The purity level was demonstrated using the size-exclusion chromatographic method for determination of "Impurities with molecular masses greater than that of insulin" described in the monograph for insulin human in the European Pharmacopoeia, 5th Edition, and using a liquid chromatography method for determination of "Related proteins" as described in the monograph for insulin aspart in the European Pharmacopoeia, 5th Edition.

Assay was determined using the latter method and quantify against a reference material of human insulin.

### Geometrical particle size, wet dispersion

The geometrical diameter was determined using a Sympatec HELOS equipped with a 6 ml SM/US cuvette. A sample of the powder was dispersed in 2-propanol and sonificated for at least three minutes prior to analysis.

### Aerodynamic particle size

The aerodynamic diameter was determined using a TSI Model 3321 Aerodynamic Particle Sizer® (APS™) spectrometer with a model 3433 Small-Scale Powder Disperser.

### Bulk density and tapped density

Bulk and Tapped density: Engelsmann tapping volumeter, A small amount of powder was weighed into a graduated cylinder and, the bulk volume was registered. Afterwards, the powder was tapped 1250 times and the tapped volume was recorded. Bulk and tapped density were calculated as well as the Carr's Index (Cl) of the insulin samples according to the following equations:
Bulk density = bulk weight / bulk volume
Tap density = bulk weight / tapped volume
Carr's Index = ((tapped density - bulk density) / tapped density) * 100

### Scanning electron microscopy (SEM)

A sample is applied to a conducting tape and coated with a gold layer of approx. 10 nm. After introduction to a vacuum chamber an incident electron beam is scanned across the surface of the sample, and the resulting electrons emitted from the sample are collected to form an image of the surface. A Zeiss Ultra55 ultra-high resolution microscope is used. Magnifications of up to 10000 times can be obtained.

### Example 1

### Spray drying of human insulin crystals

### Crystallisation:

To 4 mg of human zinc insulin crystals 25 mL of 10 mM Tris pH 8.0 with 20 % (v/v) ethanol was added. 1 N HCl was added in sufficient amount to make a clear solution, 100 mL of Tris buffer was then added and pH was adjusted to 8.0 with 1 N NaOH followed by Tris buffer up to 200 mL in total. To this solution 200 mL 2 M sodium acetate was slowly added. A precipitate was formed immediately. After two days the crystal were isolated by centrifugation at +4 °C with 3000 rpm for 20 minutes and washed with 200 mL ice cold 10 % ethanol followed by centrifugation at -10 °C with 3000 rpm for 20 minutes. The isolated wet crystal cake was stored in a refrigerator prior to further handling. Mean geometric diameter based on volume is approx. 1.6 µm.

### Spray drying:

2 grams of the wet crystal cake from above was resuspended in 80 mL ice cold ethanol, centrifuged at +4 °C with 3000 rpm for 20 minutes. 50 mL of the clear supernatant was discarded. After gentle shaking the crystal cake was suspended in the remaining 30 mL of ethanol giving a concentration of approx 13 mg/mL.

The suspension was spray dried on a Büchi Mini Spray Dryer B-290 with the following settings:

| | |
|---|---|
| Inlet temperature: | 130°C |
| Outlet temperature: | start 45 °C, end: 49 °C |
| Aspirator %: | 100 % |
| Pump %: | 6 %, corresponding to approx. 2 ml/min |
| Gas flow: | 500 normlitre/h |

During spray drying the crystal suspension was kept cold on an ice bath and stirred gently on a magnetic stirrer. The feeder and spray nozzle was cooled to approx. 4°C.

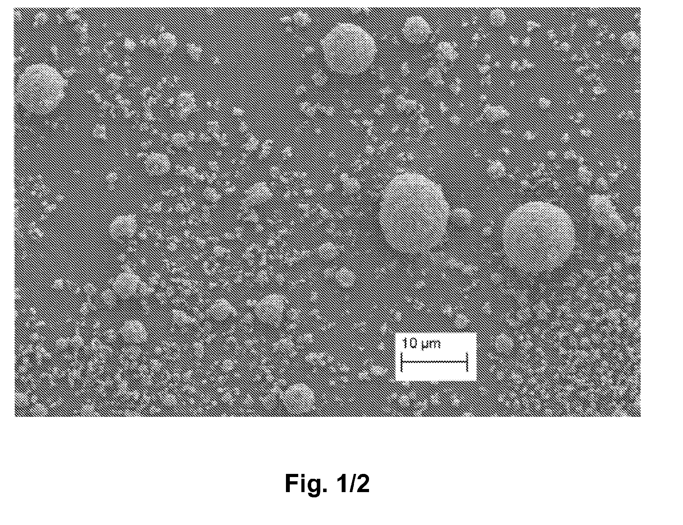

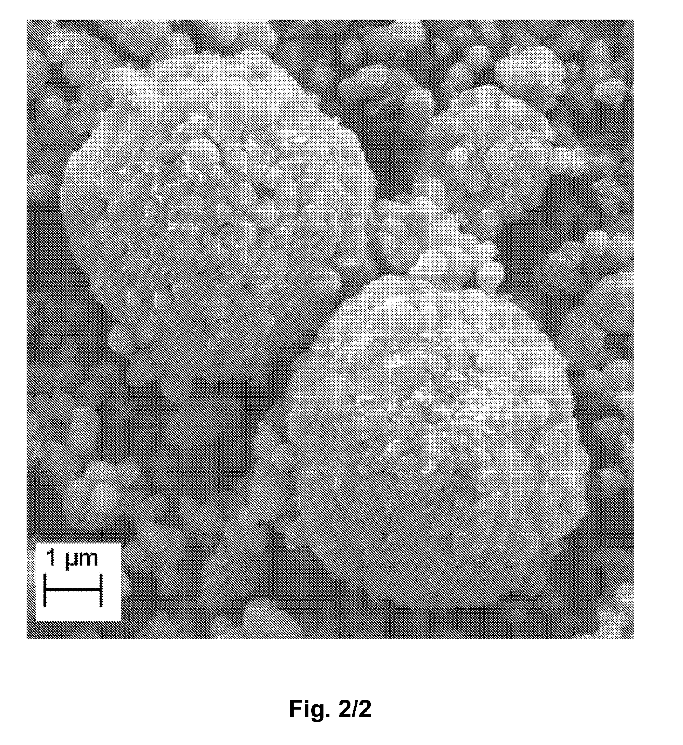

The product was isolated from the cyclone and shown in Figure 1 and Figure 2.

Analytical results for the spray dried powder are listed in Table 1.

**Table 1 List of results for spray dried powder**

| **Parameter** | **Spray dried powder** |
|---|---|
| Content (mg/mg) | 0.87 |
| Purity (%) | 97.8 |
| HMWP (%) | 0.5 |
| Geometric diameter (µm) | 4.3 |
| Aerodynamic diameter (µm) | 3.8 |

## Claims

1. A process for preparing protein particles comprising
a) Providing a suspension of protein; and
b) drying the suspension
wherein the suspension of protein is not homogenised.

2. A process according to claim 1, wherein the suspension is provided and/or fed to the drying equipment at a temperature below about 8°C, below about 6°C, below about 5°C, below about 4°C, below about 3°C, below about 2°C or below about 1°C.

3. A process according to any of the preceding claims, wherein the drying method for drying the suspension is selected from the group consisting of: spray drying, spray freeze drying, fluid bed drying and freeze drying.

4. A process according to any of the preceding claims, wherein at least 50% of the protein particles have a geometric diameter in the range of 1-100 µm.

5. A process according to any of the preceding claims, wherein at least 50% of the protein particles have a geometric diameter in the range of 1-5 µm, for example 1-3 µm.

6. A process according to claims 1-4, wherein at least 50% of the protein particles have a geometric diameter in the range of 10-50 µm, for example 10-30 µm.

7. A process according to any of the preceding claims, wherein the protein is selected from the group consisting of insulin, insulin analogues, insulin derivatives, GLP-1, GLP-1 analogues, GLP-1 derivatives, glucagon, glucagon analogues, glucagon derivatives, exendin, exendin analogues and derivatives, amylin, amylin analogues, amylin derivatives, α-MSH, α-MSH analogues, α-MSH derivatives and/or any combination thereof.

8. A process according to any of the preceding claims, wherein the suspension comprises more than one protein.

9. A process according to any of the preceding claims, wherein the suspension comprises an excipients selected from the group consisting of: a buffer, a detergent, a stabilizer, a protease inhibitor, a flavour, a carrier, an absorption protracting agent, a bulking agent or an agent improving the flowing properties or a penetration enhancer or a combination hereof.

10. A process according to any of the preceding claims, wherein the suspension liquid is water or is an organic solvent selected from the group consisting of methanol, ethanol, ethyl acetate, acetone, acetonitrile, butanol, butyl acetate, chloroform, cyclohexane, heptane, tetrahydrofuran, tetrafluoroethane and any combination thereof.

11. A process according to any of the preceding claims, wherein the protein particles has an activity of at least 40% of the activity of the suspended protein, has an activity of at least 50% of the activity of the suspended protein, at least 60% of the activity of the suspended protein, at least 65% of the activity of the suspended protein, at least 70% of the activity of the suspended protein, at least 75% of the activity of the suspended protein, at least 80% of the activity of the suspended protein, at least 85% of the activity of the suspended protein, at least 90% of the activity of the suspended protein, at least 95% of the activity of the suspended protein or at least 98% of the activity of the suspended protein.

12. A dry protein particle having a bulk density above about 0.2 g/cm³.

13. A protein particle according to claim 12 having a bulk density between about 0.2 g/cm³ and about 0.5 g/cm³.

14. Pharmaceutical composition comprising a therapeutically effective amount of a protein particle according to claims 12-13.

15. A method of treating diabetes in a patient in need of such a treatment by the use of a therapeutically effective amount of a protein particle according to claims 12-13 or a pharmaceutical composition according to claim 14.
